# EUROPEAN PATENT APPLICATION

(11) **EP 4 759 246 A1**
(43) Date of publication of application: **17.06.2026**
(21) Application number: 25223084.2
(22) Date of filing: 12.12.2025
(51) Int. Cl.: A61B 17/16

(54) **GRAFT RELEASING INSTRUMENT**

(30) Priority: 16.12.2024 US 202463734342 P; 08.12.2025 US 202519411559
(71) Applicant: Stryker European Operations Limited, Carrigtwohill, Co. Cork T45 HX08 (IE)
(72) Inventor: BOUVIER, Quentin, 38330 Montbonnot Saint Martin (FR); MAGNAC, Christophe, 38330 Montbonnot Saint Martin (FR); ZANARDI, Olivier, 38330 Montbonnot Saint Martin (FR); HENRY, Gilles, 38330 Montbonnot Saint Martin (FR)
(74) Representative: Zimmermann & Partner Patentanwälte mbB

(57) **Abstract**

A graft releasing bell instrument includes a central shaft; a bell removably coupled to the central shaft; an inner blade coupled to the central shaft; and an outer blade coupled to the bell. A method of extracting a graft of cancellous bone includes inserting the graft releasing bell instrument into a cut of a bone; confirming a depth of the cut using a gradation scale on a side of the graft releasing bell instrument; rotating the graft releasing bell instrument to cut the cancellous bone; extracting the graft releasing bell instrument from the cut; and separating the graft from the graft releasing bell instrument.

## Description

### CROSS-REFERENCE TO RELATED APPLICATION

This application claims priority to U.S. Provisional Application No. 63/734,342, filed on December 16, 2024. Any and all applications for which a foreign or domestic priority claim is identified in the Application Data Sheet as filed with the present application are hereby incorporated by reference under 37 C.F.R. § 1.57 for all purposes as if fully set forth herein.

### BACKGROUND

The present disclosure relates to surgical instruments. More specifically, the present disclosure relates to instruments for bone grafting during surgery and a method of using the instruments.

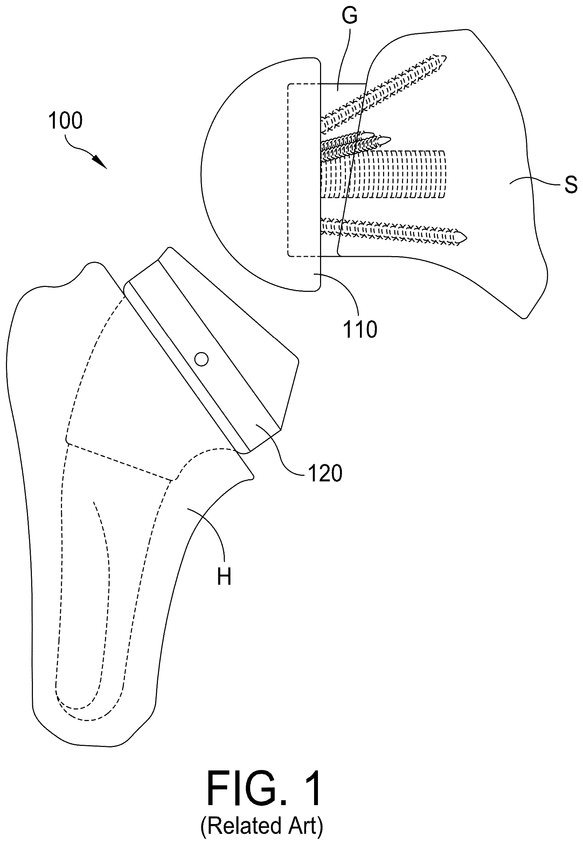

In some surgeries, it is desirable for the surgeon to harvest a portion of a bone for grafting into another location. In one example, during shoulder joint replacement, a bone graft can be taken from a humerus and be used to lateralize a glenoid implant. This might be warranted during shoulder replacement using a reverse prosthesis where a ball-shaped component is placed on the socket side (i.e., glenoid side) of the joint. This is anatomically opposite to where it is originally located. A corresponding socket-shaped component is then placed on the end of the humerus. This arrangement is known as Reverse Shoulder Arthroplasty (RSA). FIG. 1 illustrates one example of a reverse shoulder prosthesis 100 that can be used in such a procedure.

FIG. 1 shows that the reverse shoulder prosthesis 100 can include a glenoid implant 110 attached to a scapula S and a humeral implant 120 attached to a humerus H. FIG. 1 shows that a bone graft G can be used to offset or lateralize the glenoid implant 110 from the scapula S. That is, the bone graft G can be used as a spacer to shift the glenoid implant 110 laterally away from the scapula S. This can be desirable to improve stability of the prosthesis 100, reduce wear of the prosthesis 100, and increase range of shoulder motion for the patient.

The position of the glenoid implant 110 can be moved laterally to create a better foundation for the prosthetic component. This lateralization allows for better bone coverage of the prosthesis 100 and a more even distribution of forces. Bone lateralization improves the stability of the prosthesis 100, reducing the risk of dislocation. By optimizing the distribution of forces, wear on the prosthetic components can be reduced to prolong the lifespan of the prosthesis 100.

During conventional RSA, the bone graft G can be harvested from a resected humerus H. In one technique, Bony Increased Offset RSA (BIO-RSA Angled), a reamer is used prior to a graft recovery instrument to remove articular cartilage and hard bone at the head of the humerus. A saw is used with a cutting guide to resect a graft from the humerus. In another technique for a modular shoulder replacement, SMR^{®} Shoulder System, after reaming the head of the humerus, a powered graft cutter is used to cut and remove a graft. The graft is then arranged with the glenoid implant.

However, it is desirable to be minimally invasive to maintain as much bone as possible and to extract a bone cylinder from inside the humeral head without cutting off the humeral head. The current disclosure provides instruments and a method to extract a bone cylinder from inside the humeral head without cutting off the humeral head.

### SUMMARY OF THE DISCLOSURE

To overcome the problems described above, embodiments of the present disclosure include a graft releasing bell instrument comprising: a central shaft; an inner blade extending from the central shaft; an outer blade extending from an outer member; and a handle to turn the whole unit.

According to an embodiment, a graft releasing bell instrument comprises a central shaft; an inner blade extending from the central shaft; an outer blade extending from an outer member; and a handle to turn the central shaft.

In an aspect, the handle turns a gear assembly to rotate the inner blade and the outer blade.

In an aspect, the graft releasing bell instrument further comprises a button configured to be moved to different positions to extend the outer blade.

In an aspect, the graft releasing bell instrument further comprises a bell, and the bell is the outer member. A diameter of the bell is 25 mm or 29 mm.

In an aspect, the graft releasing bell instrument further comprises a graduation scale on the bell.

In an aspect, the handle is configured to fit through a hole in the central shaft.

In an aspect, the outer blade fits into a slot in a cylindrical bell.

In an aspect, the inner blade and the outer blade are rotatable to be opened and closed.

In an aspect, the central shaft is lockable to a bell.

In an aspect, the bell is configured to fit through a hole in a bone defined by a bone saw.

According to another embodiment, a method of extracting a bone graft comprises positioning a graft releasing bell instrument into a hole in a bone; opening a blade of the graft releasing bell instrument; rotating the graft releasing bell instrument to cut cancellous bone; and removing the graft releasing bell instrument and a cut portion of the cancellous bone.

In an aspect, the method can further comprise opening a second blade of the graft releasing bell instrument.

According to another embodiment, a graft releasing bell instrument comprises a body; a gear assembly coupled to the body and including a handle and gears; a first cylinder extending from the body; a first blade coupled to the first cylinder; a second cylinder extending from the body; a second blade coupled to the second cylinder; wherein rotating the handle in a first direction causes the first blade to rotate in a direction and the second blade to rotate in an opposite direction. In an aspect, there are two distinct instruments, one for obtaining a graft having a diameter of 25 mm and one for a graft having a diameter of 29 mm.

In an aspect, the first blade and the second blade are retractable within their respective cylinders.

The graft releasing bell instrument can further comprise a locking mechanism to lock and unlock the first blade and the second blade from rotating.

In an aspect, the locking mechanism includes a locking screw.

In an aspect, rotating the handle in a second direction causes the first blade and the second blade to retract.

According to another embodiment, a graft releasing bell instrument comprises an adapter; a bell coupled to the adapter; a blade coupled to the bell; and a knob coupled to the bell and the blade and configured to be movable to a plurality of discrete positions to deploy and retract the blade. In an aspect, there are different bells for different graft diameters (e.g., 25 mm and 29 mm).

In an aspect, the adapter is configured to be coupled to a power tool.

In an aspect, the adapter is configured to be coupled to a handle.

The graft releasing bell instrument can further comprise a gradation scale on a side of the bell and be configured to indicate a depth of the bell within a circular cut.

The graft releasing bell instrument can further comprise a slot across the top of the bell in which the knob can travel to deploy and retract the blade.

In an aspect, the blade is curved and conforms to an arc of the bell.

The graft releasing bell instrument can further comprise a pusher movable along a longitudinal axis of the graft releasing bell instrument to force a portion of cut bone out of the bell.

According to another embodiment, a graft releasing bell instrument comprises a central shaft; a bell removably coupled to the central shaft; an inner blade coupled to the central shaft; and an outer blade coupled to the bell.

The graft releasing bell instrument can further comprise a handle coupled to the central shaft.

In an aspect, the central shaft extends through the bell.

In an aspect, the bell is coupled to the central shaft via a pin on the central shaft fit into a T-shaped groove in a side of the bell.

In an aspect, the central shaft includes a cam, the bell includes a locking groove, and the central shaft and the bell are coupled by inserting the cam into the locking groove and rotating the central shaft and the bell with respect to each other to lock the cam in the locking groove.

In an aspect, the inner blade is located within a groove extending along a side of the central shaft and is rotatable between a deployed position and a closed position by rotating an inner blade tab connected to the inner blade.

In an aspect, the outer blade is located within a groove extending along a side of the bell and is rotatable between a deployed position and a closed position by rotating an outer blade tab connected to the outer blade.

According to another embodiment, a method of extracting a graft of cancellous bone comprises inserting the graft releasing bell instrument into a cut of a bone; confirming a depth of the cut using a gradation scale on a side of the graft releasing bell instrument; rotating the graft releasing bell instrument to cut the cancellous bone; extracting the graft releasing bell instrument from the cut; and separating the graft from the graft releasing bell instrument.

The method can further comprise cutting an opening in a humeral head prior to the inserting step.

The method can further comprise impacting the graft releasing bell instrument after the inserting step.

In an aspect, the step of rotating the graft releasing bell instrument deploys a blade of the graft releasing bell instrument.

In an aspect, the step of rotating the graft releasing bell instrument deploys two blades of the graft releasing bell instrument.

The method can further comprise deploying a blade of the graft releasing bell instrument prior to the step of rotating the graft releasing bell instrument.

The method can further comprise using the graft to lateralize a glenoid implant.

In an aspect the step of cutting the opening in the humeral head includes creating a circular opening with a bell saw.

The above and other features, elements, characteristics, steps, and advantages of the present invention will become more apparent from the following detailed description of preferred embodiments of the present invention with reference to the attached drawings.

### BRIEF DESCRIPTION OF THE DRAWINGS

FIG. 1 shows a reverse shoulder prosthesis according to related art.
FIG. 2 is a perspective view of a graft releasing instrument according to an embodiment of the present disclosure.
FIG. 3A to FIG. 3E are different views of the graft releasing instrument of FIG. 2.
FIG. 4 is a perspective view of a graft releasing instrument according to another embodiment of the present disclosure.
FIG. 5A to FIG. 5H are different views of the graft releasing instrument of FIG. 4.
FIG. 6 is a perspective view of a graft releasing instrument according to another embodiment of the present disclosure.
FIG. 7A to FIG. 7C include several views of the graft releasing instrument of FIG. 6.
FIG. 8 is a perspective view of a graft releasing instrument according to another embodiment of the present disclosure.
FIG. 9A to FIG. 9G are views of the graft releasing instrument of FIG. 8.
FIG. 10A to FIG. 10E and FIG. 11A to FIG. 11C show assembly of the shaft in the graft releasing instrument of FIG. 8.
FIG. 12A and FIG. 12B show steps of a method according to an embodiment of the present disclosure.
FIG. 13 shows a bone graft harvested from a humerus according to an embodiment of the present disclosure.

### DETAILED DESCRIPTION

Reference will now be made in detail to embodiments of the present disclosure, examples of which are illustrated in the accompanying drawings. Whenever possible, the same reference numerals will be used throughout the drawings to refer to the same or like parts. However, this disclosure may be embodied in many different forms and should not be construed as limited to the embodiments set forth herein.

Directional terms as used herein - for example up, down, right, left, front, back, top, bottom, vertical, horizontal - are made only with reference to the figures as drawn and are not intended to imply absolute orientation.

Unless otherwise expressly stated, it is in no way intended that any method set forth herein be construed as requiring that its steps be performed in a specific order, nor that with any apparatus, specific orientations be required. Accordingly, where a method claim does not actually recite an order to be followed by its steps, or that any apparatus claim does not actually recite an order or orientation to individual components, or it is not otherwise specifically stated in the claims or description that the steps are to be limited to a specific order, or that a specific order or orientation to components of an apparatus is not recited, it is in no way intended that an order or orientation be inferred, in any respect. This holds for any possible non-express basis for interpretation, including matters of logic with respect to arrangement of steps, operational flow, order of components, or orientation of components; plain meaning derived from grammatical organization or punctuation, and the number or type of embodiments described in the specification.

As used herein, the singular forms "a," "an," and "the" include plural references unless the context clearly dictates otherwise. Thus, for example, reference to "a" component includes aspects having two or more such components, unless the context clearly indicates otherwise.

In the following description, reference is made to the accompanying drawings that form a part thereof, and in which is shown by way of illustrating specific exemplary embodiments in which the disclosure may be practiced. These embodiments are described in sufficient detail to enable those skilled in the art to practice the concepts disclosed herein, and it is to be understood that modifications to the various disclosed embodiments may be made, and other embodiments may be utilized, without departing from the scope of the present disclosure. The following detailed description is, therefore, not to be taken in a limiting sense.

Disclosed graft releasing instruments, called graft releasing bells, may be used to remove a slice of bone called a "graft." In some embodiments, the graft may have a size of up to 30 millimeters in thickness, for example, and be acquired from the upper part of the humeral head. The discloses devices may be used to harvest grafts that are thicker and thinner than 30 millimeters, as will be understood by one of ordinary skill in the art. It should be understood that the disclosed devices, systems, and methods may be used with other bones, including the bones defining the hip, ankle, knee, and other joints. The graft can be cut from a bone directly from above, by inserting an instrument into a circular cut previously made with a bell saw. The diameter of the graft can be selected based on the patient's anatomy, thus limiting the number of operations and avoiding further damage to the patient's bone. The bell saw can be used to make a central hole of the graft and a circular cut corresponding to the outer diameter of the bell saw. As disclosed, the diameter and thickness of the cut graft can be selected based on the bell saw and graft releasing instrument configuration. In some embodiments, the graft can have a diameter of 25 mm or 29 mm, and the thickness for each diameter can be 20 mm, 25 mm, or 30 mm. Other suitable diameters and thicknesses are possible. Customization of the graft releasing instrument is possible to be unique or specific for one patient.

The different components of disclosed embodiments can be assembled without the need for additional tools; each assembly locks into a housing and can be used in the working position. Disassembly steps are the reverse of the assembly steps.

Conventionally, harvesting bone grafts during reverse shoulder arthroplasty can include: (i) Patient positioning: The patient can be positioned in a beach chair position with the shoulder positioned laterally to allow full arm extension; (ii) Incision: An incision can be made from the tip of the coracoid along the delto-pectoral groove; (iii) Bone preparation: Specialized reamers can be used to prepare the glenoid, creating a precise cavity for the bone graft; (iv) Graft harvesting: A cutting guide can be used to harvest a cylindrical bone graft from the humerus; and (v) Graft implantation: The harvested bone graft is then implanted into the prepared glenoid.

However, in conventional techniques, the surgeon is limited in the choice on the graft angle and the graft thickness. Instruments according to the current disclosure provide for greater choices of graft angles and thicknesses while being minimally invasive. For example, the surgeon can obtain a bone cylinder between 15 and 30 mm, and then reshape it (angle and thickness) according to their needs to fit the patient.

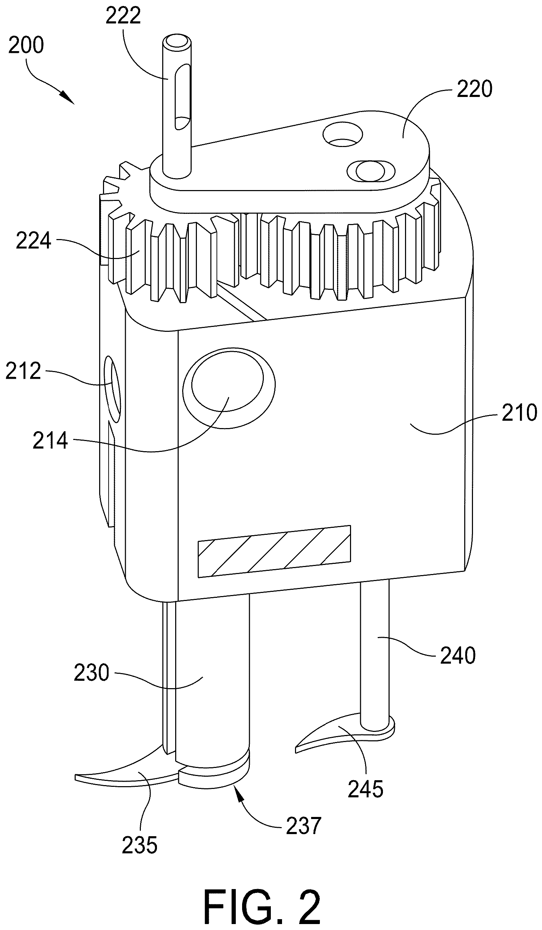

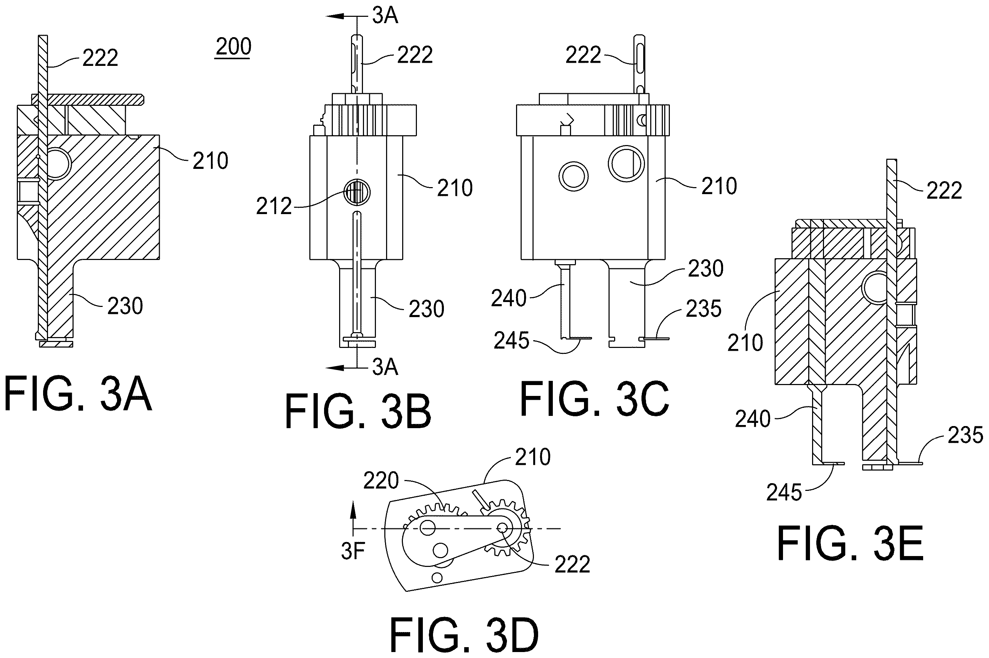

FIG. 2 is a perspective view and FIGS. 3A to 3F are drawings of a graft releasing instrument 200 according to an embodiment of the present disclosure. The drawing of FIG. 3 includes several views (3A-3F). FIG. 3B is a front view; FIG. 3A is a section view along A-A of FIG. 3B; FIG. 3C is a side view; FIGS. 3D and 3E are top views; and FIG. 3F is a section view along B-B of FIG. 3D.

As shown, the instrument 200 can include a body 210, a gear assembly 220 including a handle 222 and gears 224, a first cylinder 230, a first (internal) blade 235, a second cylinder 240, and a second (external) blade 245. The instrument 200 can be used after a head of a humerus has been opened using a bell saw.

As shown, the first cylinder 230 and the second cylinder 240 can extend from a bottom of the body 210 and the first blade 235 and the second blade 245 can extend out from their respective cylinders 230, 240. The gear assembly 220 can be located on top of the body 210 and includes a gear arrangement such that rotating the handle 222 about an axis in a first direction (e.g., a clockwise direction) rotates one of the first and second blades 235, 245 in the same direction (e.g., clockwise) about its axis of rotation and the other of the two blades 235, 245 in a second direction (e.g., counterclockwise) about its axis of rotation. In some embodiments, rotating the handle 222 and moving the gears 224 rotates shafts extending through the respective first cylinder 230 and the second cylinder 240 and connected to the respective blades 235, 245.

In some embodiments, the diameter of the first cylinder 230 can be the same diameter as a bell saw central drill (not shown). The diameter of the second blade 245 can correspond to a thickness of the bell saw walls. After drilling and reaming the humerus, with the blades 235, 245 retracted, the first cylinder 230 can be fit into the hole prepared by the central drill and then impacted in the humeral head so that the first and second cylinders 230, 240 and blades 235, 245 will be deep in the humeral head. At this depth, soft cancellous bone can be cut.

To be stored or retracted, the blades 235, 245 can be counter rotated (e.g., rotated in a second direction, such as counterclockwise) to be hidden within their respective cylinders 230, 240. For example, FIG. 2 shows the first cylinder 230 includes a slot 237 along the plane in which the first blade 235 rotates. The slot 237 can provide clearance for the first blade 235 from the first cylinder 230 while rotating 360 degrees in the cutting direction, and upon counter rotation of the gear assembly 220, for example, the first blade 235 can be effectively folded into the slot 237. A similar mechanism can be implemented for the second cylinder 240 and the second blade 245.

Additionally, in some embodiments, the first and second blades 235, 245 can be locked and unlocked for rotation. In an embodiment, the blades 235, 245 can be locked and unlocked using locking screws 212 and 214. For example, the locking screws 212, 214 can be tightened against gears or rotational members internal to the body 210 and that are attached to the blades 235, 245 to prevent those components and the respective blades from rotating. Loosening or removing the locking screws 212, 214 can permit the blades 235, 245 to rotate. In some embodiments, a pin arrangement can be used such that pressing a pin or button on the body 210 forces a pin or other component inside the body 210 to stop the gears 224 or internal member from rotating. Forcing the pin in an opposite direction can unlock the instrument 200 and permit blade rotation.

After the instrument 200 has been inserted into the hole in the humeral head and the blades 235, 245 have been unlocked and deployed, a clinician can cut the cancellous bone by holding the body 210 in one hand and rotating the handle 222 with another hand to rotate the blades 235, 245 and obtain a bone cylinder to be used as a graft. After cutting, the instrument 200 and the bone cylinder can be extracted from the humeral head, the blades 235, 245 can be retracted or closed, for example, by rotating the handle 222 in the second direction (e.g., counterclockwise), and the bone cylinder separated from the instrument 200.

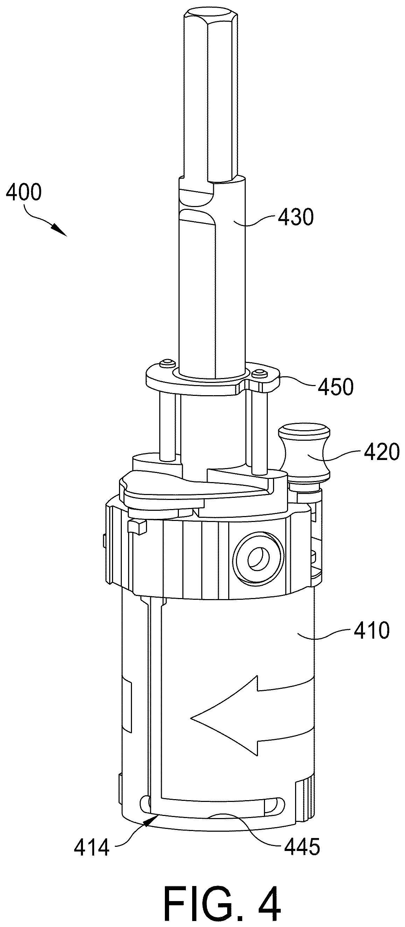

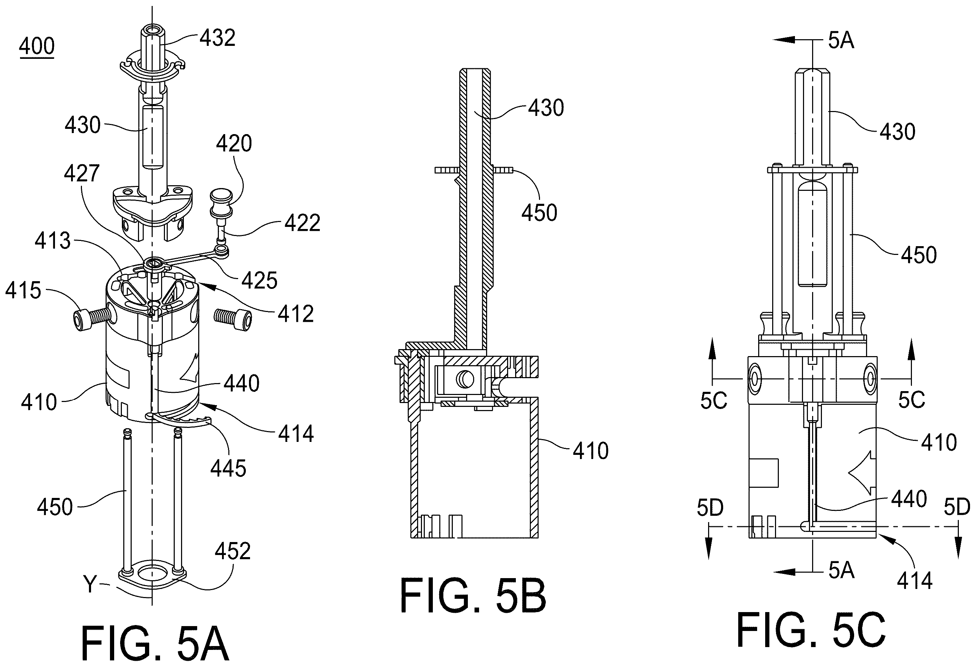

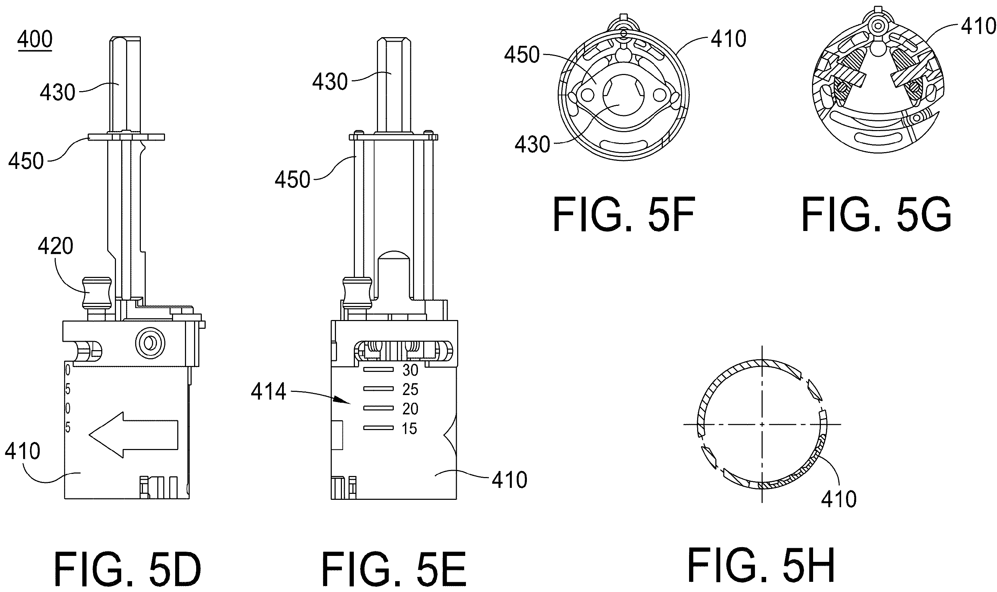

FIG. 4 is a perspective view and FIGS. 5A - 5C are drawings of a graft releasing instrument 400 according to another embodiment of the present disclosure. The drawing of FIG. 5 includes several views5A-5H. FIG. 5A is an exploded view; FIG. 5C is a front view; FIG. 5B is a section view along A-A of FIG. 5C; FIG. 5D is a side view; FIG. 5E is a rear view; FIG. 5F is a top view; FIG. 5G is a section view along C-C of FIG. 5C; and FIG. 5H is a section view along D-D of FIG. 5C. As shown, the instrument 400 can include a bell 410, fasteners 415, a knob 420, a knob connector 425, an adapter 430, a blade shaft 440, a blade 445, and a pusher 450. The bell 410 can be hollow and connected to the adapter 430 using the fasteners 415. The bell 410 can be interchangeable with other bells having different diameters. In some embodiments, the bell 410 can have a 25 mm diameter. In other embodiments, the bell 410 can have a 29 mm diameter. Optionally, the bell 410 can have any suitable diameter.

The adapter 430 can be elongated and can be configured to be connected to a power tool (not shown) used to rotate the instrument 400 about a central axis Y, shown in FIG. 5A. For example, a free end 432 of the adapter 430 can be fit into a chuck or collet of the power tool. In some embodiments, a handle can be attached to the adapter 430 and used to rotate the instrument 400 by hand.

The knob 420 can be used to manually deploy (e.g., radially outwardly extend) the blade 445 from its collapsed position in which the blade is stored within a slot 414 defined by the wall of the bell 410. As shown in FIG. 5A, the slot 414 can be arched to extend a portion of a circumference of the wall of the bell 410. FIG. 5A also shows that the knob 420 can be connected to the blade 445 via an arm 425 extending between the knob 420 and a centrally located pivot 427. In turn, the pivot 427 can be connected to the blade shaft 440 extending vertically through the bell to the blade 445. To deploy the blade 445, the knob 420 can be manually moved to successive positions or detents 413 located along an arc-shaped slot 412 that extends across the top of the bell 410 to stepwise rotate the blade 445 inside the bell 410. The detents 413 can be defined as suitably shaped cutouts along the slot 412. The arm 425 can be elastic and allow the blade 445 to move against the bone during position changes of the knob 420. In some embodiments, the blade 445 is curved and conforms to an arc of the bell 410 such that the blade 445 can be stored within the slot 414 along a wall of the bell 410. In some embodiments, the blade 445 can include cutting teeth.

The instrument 400 can be used after a head of a humerus has been opened using a bell saw. In use, the instrument 400 can be introduced into the circular cut in a humerus made by the bell saw, until it rests on the bottom of the cut. The depth can be checked by using graduations 414 on the bell 410, visible in FIG. 5E. The graduations 414 indicate the depth of the cut made by the bell saw, for example, 15 mm, 20 mm, 25 mm, and 30 mm.

The blade 445 can be manipulated using the knob 420 by moving the knob 420 to the multiple detents 413 along the slot 412. While the instrument 400 is not rotating, to avoid breaking any components, the knob 420 can be pulled up, moved to another detent 413 while rotating the blade 445 inward to pre-stress the blade 445 (with cutting teeth) against the bone, and pushed back into a position in the slot 412. The force of the blade 445 against the bone causes the blade 445 to cut the bone within several rotations in each position. In some embodiments, there are four detents 413, but any other suitable amounts are possible.

Using the power tool or handle attached to the adapter 430, the surgeon can turn the instrument 400 clockwise any number of turns for each knob/blade position. The number of turns can vary based on the rotating speed and surgical conditions. For example, the number of turns can be about four. After cutting in one position, the surgeon can move the knob 420 to the next detent 413 to further extend the blade 445. The knob 420 can include a top portion that the surgeon can grip and a shaft 422 extending from the top portion through the knob connector 425 and into the slot 412 or a detent 413. After moving through all different knob detents 413, the blade 440 is entirely open and in position to complete a cut of cancellous bone.

Before removing the cut graft, the blade 445 should be closed to not interfere with extraction of the graft from the bell 410. The blade 445 can be closed by moving the knob 420 to the starting position and fully retracting the blade 445. If the graft bone is caught in the bell 410, the pusher 450 including a plate 452 can be pushed down along the Y axis to force the graft outside the bell 410.

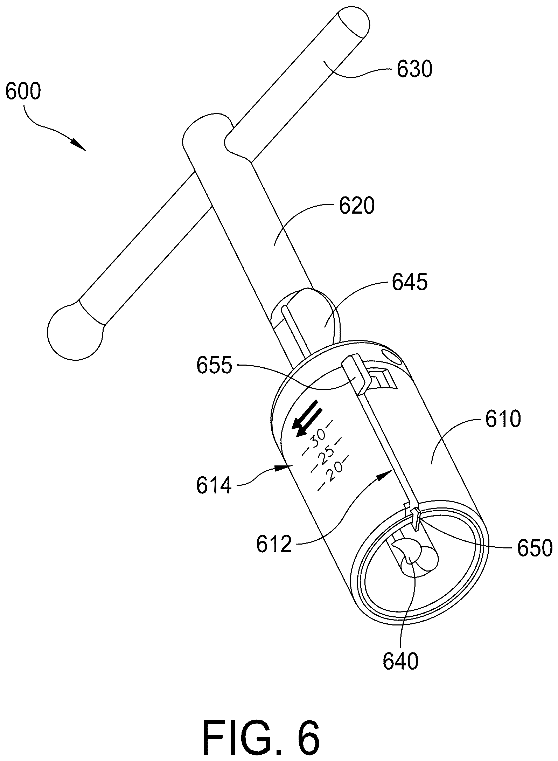

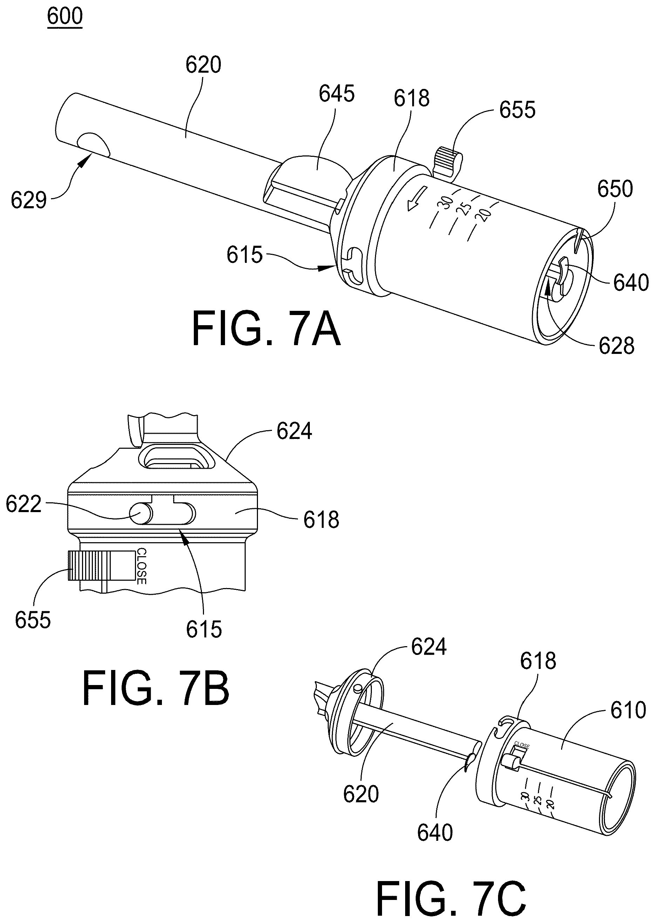

FIG. 6 is a perspective view of a graft releasing bell instrument 600 according to another embodiment of the present disclosure. FIGS. 7A -7C include several views of the instrument 600. FIG. 7A is a different perspective view than FI, FIG. 7B is a side view of a connecting portion, and FIG. 7C is a partial exploded view of the graft releasing instrument 600.

As shown in FIGS. 6 and 7A-7C, the instrument 600 can include a bell 610, a central shaft 620, a handle 630, an inner blade 640, and an outer blade 650. The bell 610 can be removable and interchanged with other bells with different diameters. In some embodiments, the bell 610 can have a 25 mm diameter. In other embodiments, the bell 610 can have a 29 mm diameter. The bell 610 can have any suitable diameter. The instrument 600 can be used after a head of a humerus has been opened using a bell saw.

As shown in FIG. 7B, prior to use, the chosen bell 610 can be locked on the central shaft 620 via a locking inverted T-shaped groove 615 in a top portion 618 of the bell 610. A dome-shaped bell cap 624 can be attached to the central shaft 620 and can include a pin 622 that protrudes from a side. For coupling, a top portion 618 of the bell 610 having a greater diameter can be aligned to the bell cap 624 such that the pin 622 fits through an opening slot in the T-shaped groove 615 before rotating the bell 610 such that the pin 622 is locked with the T-shaped groove 615. There can be more than one T-shaped groove 615 and corresponding pin 622.

The inner blade 640 can be inserted on the central shaft 620 by sliding it through a central shaft groove 628 until the final position of the inner blade 640. The inner blade 640 can be rotatable between a deployed position and a closed position by rotating an inner blade tab 645 connected to the inner blade 640. The outer blade 650 can be inserted on the bell 610 by sliding its shaft through a groove 612 extending vertically along the side of the bell 610 until the final position of the outer blade 650. The outer blade 650 can be rotatable between a deployed position and a closed position by rotating an outer blade tab 655 connected to the outer blade 650. The handle 630 can be inserted into a hole 629 through the central shaft 620.

In use, the instrument 600 can be introduced into the circular cut in a humerus made by the bell saw, until it rests on the bottom of the cut. Cutting depth can be checked by using graduations and scale 614 on the bell 610 (e.g. 20mm, 25mm, or 30mm). Turning the instrument 600 clockwise via the handle 630 rotates the blades 640, 650 into the "open" position after initiating the blades rotation by hand with the outer blade tab 655 connected to the outer blade 650 and the inner blade tab 645 connected to the inner blade 640. Cutting resistance forces the blades 640, 650 to their fully open position automatically during the instrument 600 rotation. Continually rotating the instrument 600 cuts the cancellous bone to define a graft. After the graft is cut, the instrument 600 is withdrawn from the bone, while continuing to turn slightly clockwise. The graft will remain inside the bell 610 because the blades 640, 650 are in the "open" or deployed position at the bottom of the graft. The blades 640, 650 can be moved to the "closed" or retracted position by moving their respective tabs 645, 655. The bone graft can then be extracted from the bell 610 with the blades 645, 655 rotated out of the way.

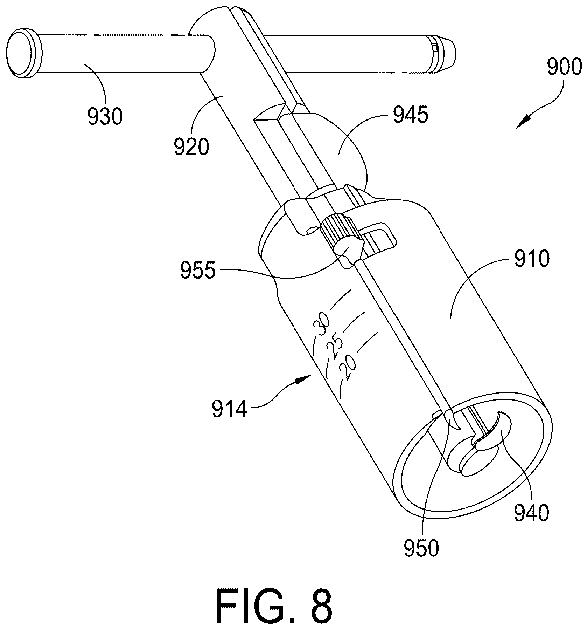

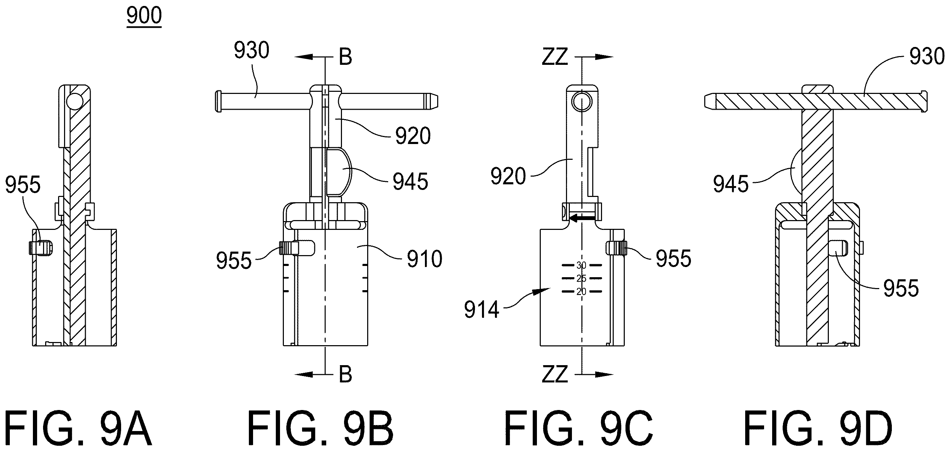

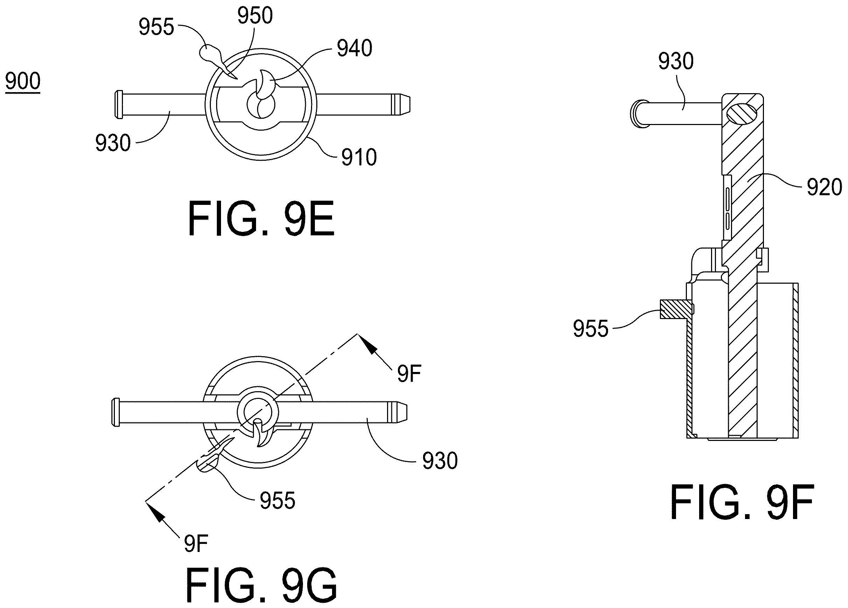

FIG. 8 is a perspective view of a graft releasing instrument 900 according to another embodiment of the present disclosure. FIGS. 9A-9G include several views of the instrument 900. The drawing of FIG. 9 includes several views 9A-9G. FIG. 9B is a front view; FIG. 9A is a section view along B-B of FIG. 9B; FIG. 9C is a side view; FIG. 9D is a section view along ZZ-ZZ of FIG. 9C; FIG.9E is a bottom view; FIG. 9F is a perspective section view taken along Y-Y of FIG. 9G; and FIG. 9G is a top view. The graft releasing instrument 900 can include features like the instrument 600 descriptions of which are omitted for brevity.

As shown, the graft releasing instrument 900 can include a bell 910, a central shaft 920 extending through the bell 910, a handle 930 attached to and defining a T-shape with the central shaft 920, an inner blade 940 extending from one end of the central shaft 920 that is in the bell 910, and an outer blade 950 extending inward from a perimeter of the bell 910. The bell 910 can be interchangeable with other bells having different diameters. In some embodiments, the bell 910 can have a 25 mm diameter. In other embodiments, the bell 910 can have a 29 mm diameter. The bell 910 can have any suitable diameter. The instrument 900 can be used after a head of a humerus has been opened using a bell saw as described with respect to the use of the instrument 600.

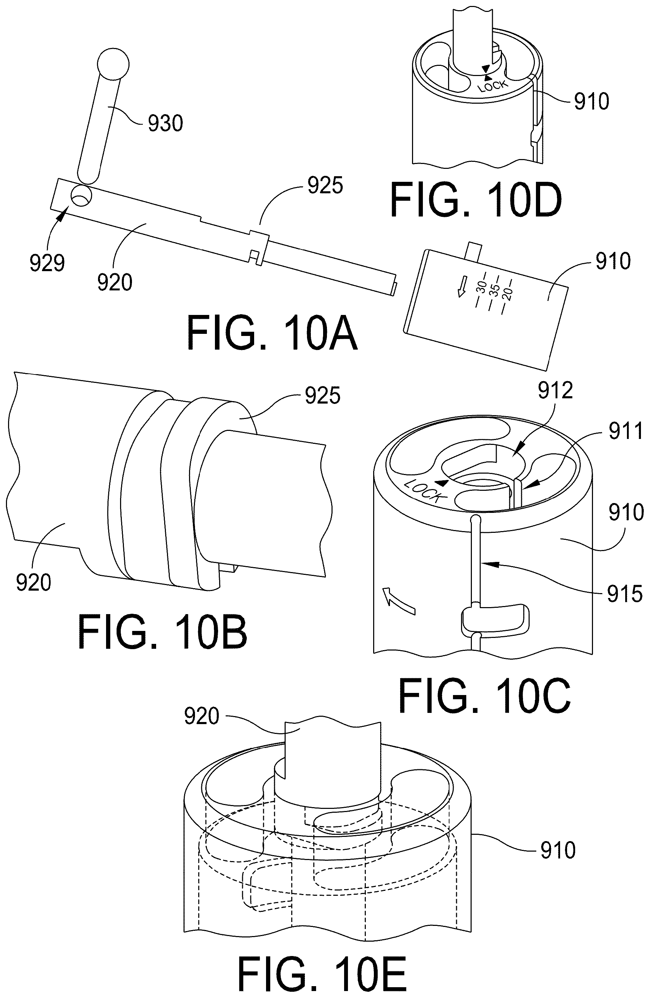

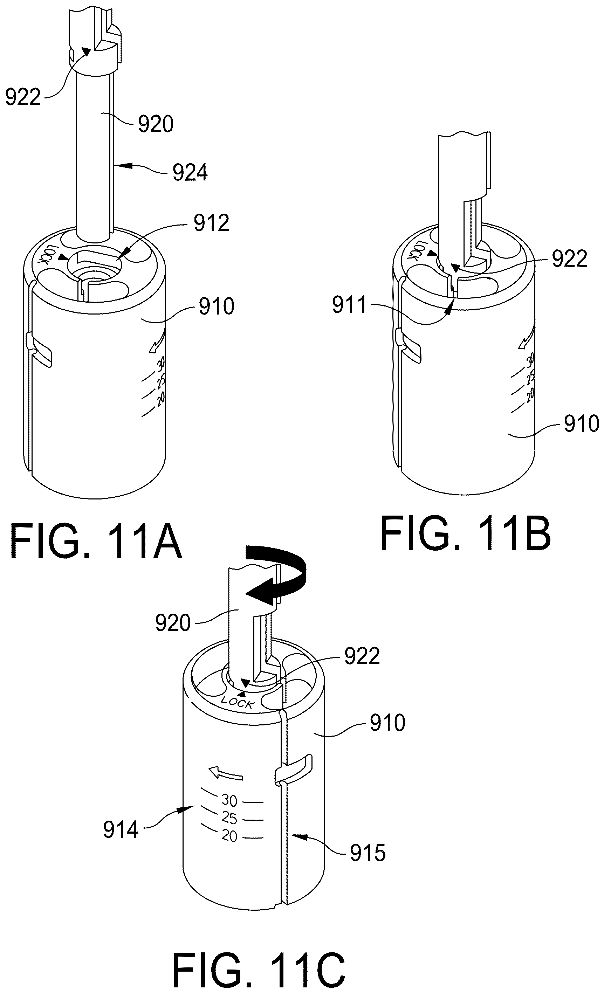

Prior to cutting a bone graft, the instrument 900 can be assembled starting with the bell 910, the central shaft 910, and the handle 930, as shown in FIG. 10A and FIG. 11A. Rather than the mechanism of the inverted T-shaped groove 615 and pin 622 of the instrument 600, the bell 910 with the appropriately size can be locked on the central shaft 920 via a locking cam 925, shown in the close-up view of FIG. 10B, interfaced in a locking groove 912 of the bell 910. First, the central shaft 920 can be inserted into the chosen bell 910, matching an alignment indicator 922 (e.g., shown as an arrowhead in FIGS. 11A-11C) on the central shaft 920 to an "unlock" indicator like a slot 911, for example, as shown in FIG. 10C and FIG. 11B. Once the central shaft 920 is in position in the bell 910, a rotation (e.g., 1/4 turn) is made to lock the central shaft 920 in the bell 910 to align the alignment indicator 922 with a "lock" marking, for example, as shown in FIG. 10D and FIG. 11C. FIGS. 10E and 11C are views showing the arrangement of aligned indicator markings such that the central shaft 920 and the bell 910 are locked together.

The inner blade 940 is inserted on the central shaft 920 by sliding it through a central shaft groove 924 that extends vertically at least down a portion of the central shaft 920 until the final position of the inner blade 940. The inner blade 940 is placed in the "closed" position by rotating an inner blade tab 945 connected to the inner blade 940. The outer blade 950 is inserted on the bell 910 by sliding its shaft through a bell groove 915 until the final position of the outer blade 950. The outer blade 950 is placed in the "closed" position by rotating an outer blade tab 955 connected to the outer blade 950. The handle 930 is inserted into a hole 929 through the central shaft 920.

Like the instrument 600, in use, the instrument 900 is introduced into the circular cut in a humerus made by the bell saw, until it rests on the bottom of the cut. Cutting depth can be checked by using graduations 914 on the bell 910 (e.g. 20mm, 25mm, or 30mm). Turning the instrument 900 clockwise via the handle 930 rotates the blades 940, 950 into the "open" position after initiating the blades rotation by hand with the outer blade tab 955 connected to the outer blade 950 and the inner blade tab 945 connected to the inner blade 940. Cutting resistance forces the blades 940, 950 to their fully open position automatically during the instrument 900 rotation. Continually rotating the instrument 900 cuts the cancellous bone to define a graft. After the graft is cut, the instrument 900 is withdrawn from the bone, while continuing to turn slightly clockwise. The graft will remain inside the bell 910 because the blades 940, 950 are in the "open" position. The blades 940, 950 are moved to the "closed" position by moving their respective tabs 945, 955. The handle 930 and the blades 940, 950 can be removed from the instrument 900 before the bone graft is extracted from the bell 910. The bone graft can be removed from the bell 910 using any suitable technique including using a graft extractor and/or by vertical impaction to the instrument 900 or the bell 910.

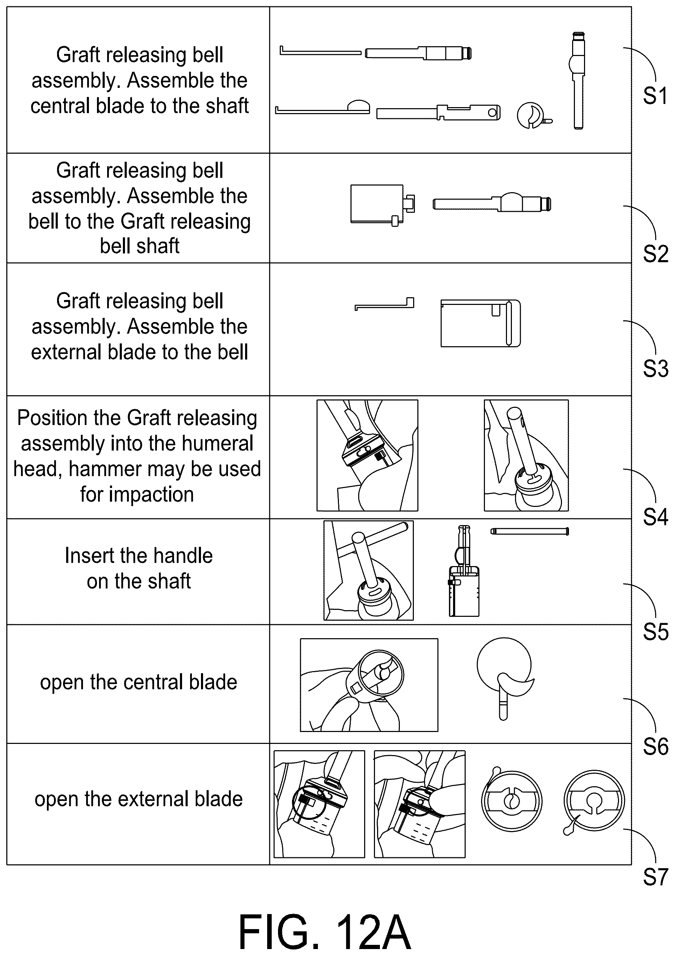

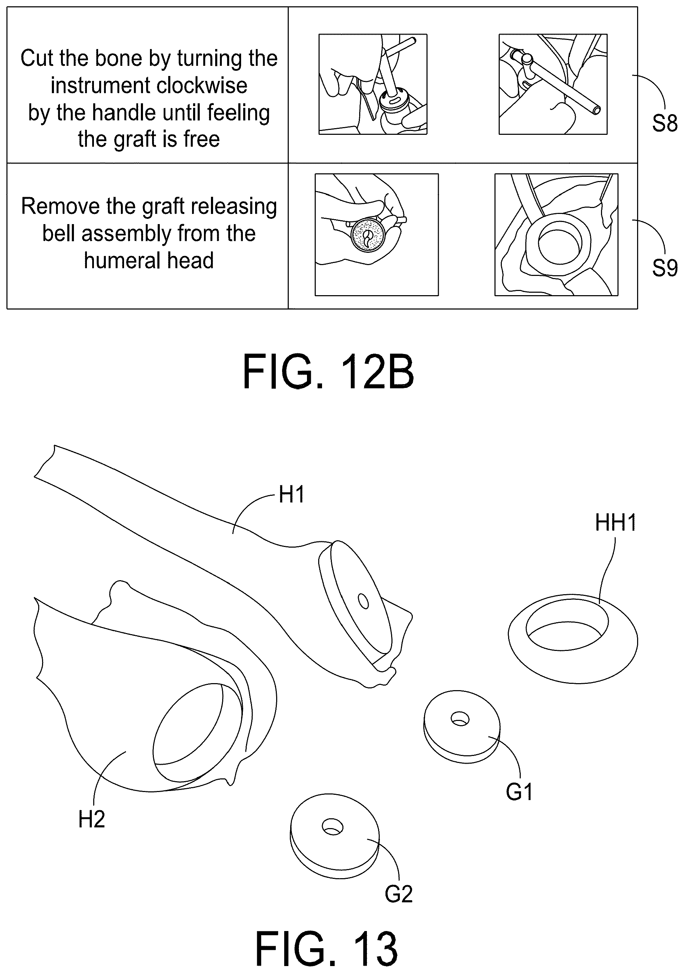

FIGS. 12A and 12B describe steps of a method 1200 of extracting a graft of cancellous bone from a humerus according to an embodiment of the present disclosure.

The method 1200 begins by assembling a graft releasing bell instrument. In step S1, an inner blade can be assembled to a central shaft. In step S2, a bell can be assembled to the central shaft. In step S3, an outer blade can be assembled to the bell.

In step S4, the bell of the graft releasing bell instrument can be placed into an opening in a humeral head that has been cut with a bell saw. A hammer or other tool can be used to impact the central shaft of the graft releasing bell instrument into the opening in the humeral head to engage the instrument with the cancellous bone at the bottom of the cut made by the bell saw. The depth can be checked using graduations on the bell. In step S5, a handle can be inserted through the central shaft. In step S6, an inner blade of the graft releasing bell instrument is extended or opened. In step S7, an outer blade of the graft releasing bell instrument can be extended or opened.

In step S8 of FIG. 12B, the graft releasing bell instrument can be turned, e.g. clockwise, to cut the cancellous bone. The graft releasing bell instrument can be turned until a cylindrical portion of the cancellous bone is cut free from the humerus. In step S9, the graft releasing bell instrument along with the cut portion of the cancellous bone is removed from the humeral head. The cut portion of the cancellous bone can be separated from the graft releasing bell instrument, inspected, sized, and shaped to be available to use as a graft to lateralize a glenoid implant, for example.

As illustrated in FIG. 13, the characteristics and features of the disclosed instruments including two blades and method permit the surgeon can extract a bone cylinder graft from inside the humeral head as compared to shearing off the head. At the top portion of FIG. 13, there is shown results of the BIO RSA technique where the surgeon had to cut the humeral head HH1 from the humerus H1 to remove the bone graft G1. At the bottom portion of FIG. 13, using the instruments and method according to the present disclosure, the humeral head does not have to be entirely cut to remove the bone graft G2 from the humerus H2. Instead, a bell saw can be used to access the head of humerus H2. The surgeon can obtain a bone graft cylinder G2 between 15 mm and 30 mm thick, for example. After the bone graft G2 is removed, RSA techniques that lateralize the glenoid implant can be used.

Further to the above, embodiments according to the following clauses are disclosed herein.

Clause 1. A graft releasing bell instrument comprising:
a central shaft;
an inner blade extending from the central shaft;
an outer blade extending from an outer member; and
a handle to turn the central shaft.

Clause 2. The instrument of clause 1, further comprising a button configured to be moved to different positions to extend the outer blade.

Clause 3. The instrument of clause 1, further comprising a bell.

Clause 4. The instrument of clause 3, wherein the bell is the outer member.

Clause 5. The instrument of clause 1, wherein the handle is configured to fit through a hole in the central shaft.

Clause 6. The instrument of clause 3, wherein a diameter of the bell is 25 mm or 29 mm.

Clause 7. The instrument of clause 3, further comprising a graduation scale on the bell.

Clause 8. The instrument of clause 1, wherein the outer blade fits into a slot in a cylindrical bell.

Clause 9. The instrument of clause 3, wherein the outer blade fits into a slot in the bell.

Clause 10. The instrument of clause 1, wherein the inner blade and the outer blade are rotatable to be opened and closed.

Clause 11. The instrument of clause 1, wherein the central shaft is lockable to a bell.

Clause 12. The instrument of clause 3, wherein the bell is configured to fit through a hole in a bone defined by a bone saw.

Clause 13. A graft releasing bell instrument comprising:
a central shaft;
a bell removably coupled to the central shaft;
an inner blade coupled to the central shaft; and
an outer blade coupled to the bell.

Clause 14. The graft releasing bell instrument of clause 13 further comprising, a handle coupled to the central shaft.

Clause 15. The graft releasing bell instrument of clause 13, wherein the central shaft extends through the bell.

Clause 16. The graft releasing bell instrument of clause 13, wherein
the central shaft includes a cam,
the bell includes a locking groove, and
the central shaft and the bell are coupled by inserting the cam into the locking groove and rotating the central shaft and the bell with respect to each other to lock the cam in the locking groove.

Clause 17. The graft releasing bell instrument of clause 13, wherein a diameter of the bell is one of 25 mm and 29 mm.

Clause 18. The graft releasing bell instrument of clause 13 further comprising, a gradation scale on a side of the bell and configured to indicate a depth of the bell within a circular cut.

Clause 19. The graft releasing bell instrument of clause 13, wherein the inner blade is located within a groove extending along a side of the central shaft and is rotatable between a deployed position and a closed position by rotating an inner blade tab connected to the inner blade.

Clause 20. The graft releasing bell instrument of clause 13, wherein the outer blade is located within a groove extending along a side of the bell and is rotatable between a deployed position and a closed position by rotating an outer blade tab connected to the outer blade.

It should be understood that the foregoing description is only illustrative of the present invention. Various alternatives and modifications can be devised by those skilled in the art without departing from the present invention. Accordingly, the present invention is intended to embrace all such alternatives, modifications, and variances that fall within the scope of the appended claims.

## Claims

1. A graft releasing bell instrument comprising:
a central shaft;
an inner blade extending from the central shaft;
an outer blade extending from an outer member; and
a handle to turn the central shaft.

2. The instrument of claim 1, further comprising a button configured to be moved to different positions to extend the outer blade.

3. The instrument of claim 1 or 2, further comprising a bell.

4. The instrument of claim 3, wherein the bell is the outer member.

5. The instrument of any of the preceding claims, wherein the handle is configured to fit through a hole in the central shaft.

6. The instrument of any of claims 3 to 5, wherein a diameter of the bell is 25 mm or 29 mm.

7. The instrument of any of claims 3 to 6, further comprising a graduation scale on the bell.

8. The instrument of any of the preceding claims, wherein the outer blade fits into a slot in a bell, in particular wherein the outer blade is located within a groove extending along a side of the bell and is rotatable between a deployed position and a closed position by rotating an outer blade tab connected to the outer blade.

9. The instrument of claim 8, wherein the the bell is cylindrical.

10. The instrument of the preceding claims, wherein the inner blade and the outer blade are rotatable to be opened and closed.

11. The instrument of any of the preceding claims, wherein the central shaft is lockable to a bell, in particular wherein the bell is removably coupled to the central shaft.

12. The instrument of claim 11, wherein
the central shaft includes a cam,
the bell includes a locking groove, and
the central shaft and the bell are coupled by inserting the cam into the locking groove and rotating the central shaft and the bell with respect to each other to lock the cam in the locking groove.

13. The instrument of any of the preceding claims, wherein the inner blade is located within a groove extending along a side of the central shaft and is rotatable between a deployed position and a closed position by rotating an inner blade tab connected to the inner blade.

14. The instrument of any of claims 3 to 13, wherein the bell is configured to fit through a hole in a bone defined by a bone saw.
